# EUROPEAN PATENT APPLICATION

(11) **EP 0 953 359 A1**
(43) Date of publication of application: **03.11.1999**
(21) Application number: 97949114.9
(22) Date of filing: 17.12.1997
(51) Int. Cl.: A61K 47/40

(54) **PHARMACEUTICAL COMPOSITION FOR ORAL ADMINISTRATION**

(30) Priority: 19.12.1996 JP 33963896
(71) Applicant: DAIICHI PHARMACEUTICAL CO., LTD., Chuo-ku, Tokyo 103 (JP)
(72) Inventor: TAKAHASHI, Masayuki, Daiichi Pharmaceut. Co., Ltd., Tokyo 134 (JP); MORITA, Hiromi, Daiichi Pharmaceutical Co., Ltd., Tokyo 134 (JP); KIKUCHI, Hiroshi, Daiichi Pharmaceutical Co., Ltd., Tokyo 134 (JP)
(74) Representative: Wächtershäuser, Günter, Prof. Dr.
(86) International application number: JP9704650
(87) International publication number: WO9826803

(57) **Abstract**

The present invention is directed to a pharmaceutical composition for oral administration, comprising a basic drug, a lipophilic substance, and/or a cyclodextrin.

The composition exhibits improved peroral absorption of a basic drug which is difficult to be absorbed by oral administration.

## Description

### Technical Field

The present invention relates to a pharmaceutical composition exhibiting improved peroral absorption of a basic drug which is considered less likely to be absorbed by oral administration.

### Background Art

Among a variety of basic drugs, some do not provide pharmacological effects through peroral administration because of their low absorption rate in the digestive tract.

Conventionally, such drugs exhibiting low absorption through peroral administration have been administered intravenously or intramuscularly. Administration by way of injection involves various problems such as pain, muscle injury, and complicated operations.

To solve these problems, various additives (absorption enhancer) for promoting absorption rate have been suggested. However such additives may cause dysfunction of the mucosa. Also, they may invite additional problems such as absorption of physiologically unnecessary components, because of the increase in absorption rate through digestive tract membranes which serve as a biological barrier, and absorption of the additives themselves, raising concerns in relation to safety.

Cyclodextrin is a non-reducing cyclic malto-oligosaccharide having 6-12 glucose molecules in the form of a ring, and is known to trap various chemical compounds in its molecular cavity, thereby forming an inclusion complex Cyclodextrin has been reported to improve peroral absorption of a compound, but this is true only in the case in which the compound is included in the molecular cavity of cyclodextrin. Generally, a basic compound contains a polar group such as an amino group in the molecule and for this reason is not included in the cyclodextrin molecular cavity.

As described above, existing techniques have failed to establish a safe method for providing efficient absorption of a basic drug having a low oral bioavailability.

The present invention is directed to solving the problems of such existing techniques, and aims at safely improving the oral bioavailability of basic drugs which fail to provide expected pharmacological effect through peroral administration because of poor absorption in the digestive tract.

### Disclosure of the Invention

The present inventors have conducted extensive studies in an attempt to improve the oral bioavailability of a basic drug which does not provide expected pharmacological effect by peroal administration. As a result, the present inventors found that oral bioavailability is improved by the addition of a lipophilic substance such as an ester formed between fatty acid and glycerin, and/or a cyclodextrin, which have been widely used as foods and pharmaceuticals and confirmed to be safe, thus leading to completion of the present invention.

Accordingly, the present invention provides a pharmaceutical composition for oral administration which comprises (a) a basic drug and (b) a lipophilic substance and/or a cyclodextrin.

### Best Modes for Carrying Out the Invention

In the present invention, the expression "basic drug" refers to a drug whose chemical structure contains one or more basic groups such as an amino group or an amidino group, a salt thereof, or a solvate of the drug itself or the salt. The position at which the basic group is located is not particularly limited. For example, the basic group may be located in the center of the molecule or its vicinity, or at a terminal or its vicinity, or basic groups may be located at both terminals or their vicinities.

Such basic drugs are not specifically limited, so long as they contain the above-described basic groups. Examples of such basic drugs include basic extracted materials (extracts, tinctures, etc.) derived from natural crude drugs, basic compounds isolated from the extracts or similar materials, and chemically synthesized basic compounds. The compounds may comprise a single component or mixture of two or more components.

When the basic drug contains an asymmetric carbon atom, and optical isomers or stereoisomers exist, any one of such optical isomers or stereoisomers or a mixture thereof may be encompassed by the basic drugs as defined by the present invention.

Salts of the basic drugs are not particularly limited so long as they are pharmaceutically acceptable, and specific examples include salts of mineral acids such as hydrochloric acid, hydrobromic acid, hydroiodic acid, phosphoric acid, nitric acid, and sulfuric acid; salts of organic sulfonic acids such as methanesulfonic acid, 2-hydroxyethanesulfonic acid, and p-toluenesulfonic acid; and salts of organic carboxylic acids such as acetic acid, propionic acid, oxalic acid, malonic acid, succinic acid, glutaric acid, adipic acid, tartaric acid, maleic acid, malic acid, and mandelic acid.

Any solvates of basic drugs or solvates of salts of the basic drugs may be used so long as they are pharmaceutically acceptable. Among them, hydrates are preferred.

In the present invention, basic drugs are not particularly limited. For example, when basic drugs which exhibit low oral bioavailability through the digestive tract and yet provide sufficient pharmacological effect through oral administration are used, dosage of the drugs can be reduced by enhancing a oral bioavailability, to thereby reduce adverse side effects. When a basic drug that does not provide sufficient pharmaceutical effect due to a low oral bioavailability is used, the drug can provide expected pharmacological effects by enhancing the oral bioavailability. In general, drugs having a low oral bioavailability are highly hydrophilic.

Examples of the basic drugs which are used in the present invention include aromatic amidine derivatives, salts of the derivatives, solvates of the derivatives, and solvates of salts of the derivatives. Aromatic amidine derivatives are encompassed by the aforementioned basic compounds, and refer to compounds having an aromatic amidine structure. Specific examples of such basic drugs include derivatives as described in Japanese Patent Application Laid-Open (*kokai*) No. 5-208946 and International Patent Publication WO 96/16940, which are represented by the following formula (1), salts thereof, solvates of the derivatives, and solvates of the salts of the derivatives: 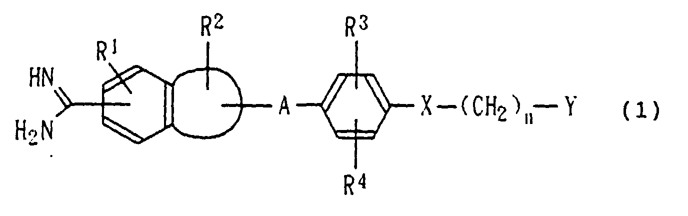
[wherein R¹ represents a hydrogen atom or a lower alkoxyl group;
R² represents a hydrogen atom, a lower alkyl group, a lower alkoxyl group, a carboxyl group, an alkoxycarbonyl group, a carboxyalkyl group, or an alkoxycarbonylalkyl group;
R³ represents a hydrogen atom, a carboxyl group, an alkoxycarbonyl group, a carboxyalkyl group, an alkoxycarbonylalkyl group, a carboxyalkoxyl group or an alkoxycarbonylalkoxyl group;
R⁴ represents a hydrogen atom, a halogen atom, an amino group, a cyano group, a nitro group, a hydroxyl group, a lower alkyl group, or a lower alkoxyl group;
n represents an integer 0 to 4; and
A represents a C1-C4 alkylene group optionally substituted by one or two hydroxyalkyl groups, carboxyl groups, alkoxycarbonyl groups, carboxyalkyl groups, or alkoxycarbonylalkyl groups, or a group represented by the following formula; 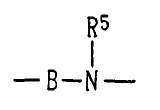
{wherein B represents a lower alkylene group or a carbonyl group and R⁵ represents a hydrogen atom or a group represented by formula -D-W-R⁶ (wherein D is a group represented by 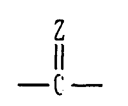
(wherein Z is an oxygen atom or a sulfur atom), or a group represented by 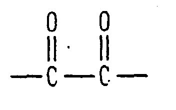 or a sulfonyl group;
W represents a single bond or a group represented by -NR⁷-(wherein R⁷ represents a hydrogen atom, a carbamoyl group, a lower alkoxycarbonyl group, a mono- or di-lower alkylaminocarbonyl group, a lower alkylsulfonyl group, a mono- or di-lower alkylaminothiocarbonyl group, a lower alkyl group which may have a substituent, or a lower alkanoyl group which may have a substituent);
R⁶ represents a hydroxyl group, a lower alkoxyl group, a lower alkyl group which may have a substituent, an aryl group which may have a substituent, or a heteroaryl group which may have a substituent)};
X represents a single bond, an oxygen atom, a sulfur atom, or a carbonyl group;
Y represents a saturated or unsaturated 5- or 6-membered heterocyclic or cyclic hydrocarbon group which may have a substituent, an amino group which may have a substituent, or an aminoalkyl group which may have a substituent; and the group represented by 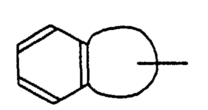 represents a group selected from among indolyl, benzofuranyl, benzothienyl, benzimidazolyl, benzoxazolyl, benzothiazolyl, naphthyl, tetrahydronaphthyl, or indanyl]; and the following compounds.

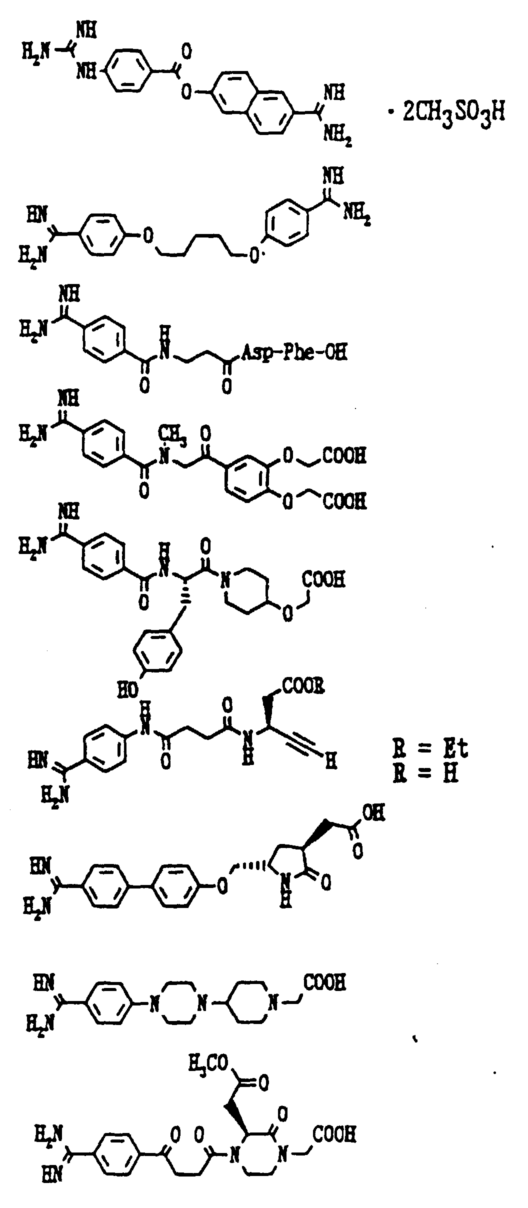

These compounds are already known and can be prepared by a known production method.

Of these, there are preferred as the basic drugs derivatives of formula (1), salts of the derivatives, solvates of the derivatives, and solvates of the salts of the derivatives.

In the above-described formula (1), examples of the lower alkyl group include C1-C6 linear, branched, and cyclic alkyl groups. Specific examples thereof include a methyl group, an ethyl group, a propyl group, an isopropyl group, a butyl group, a secondary butyl group, a tertiary butyl group, a pentyl group, a hexyl group, a cyclopropyl group, a cyclobutyl group, a cyclopentyl group, and a cyclohexyl group.

The lower alkyl group may have a substituent, and examples of the substituent include a halogen atom, a carboxyl group, a carbamoyl group, an amino group, a cyano group, a nitro group, a lower alkanoyl group, a lower alkoxyl group, a lower alkoxycarbonyl group, a mono- or di- lower alkylamino group, an aryl group, an aralkyloxy group, an aryloxy group, a mercapto group, a lower alkylthio group, a lower alkylthiocarbonyl group, a hydroxyl group, a carbamoyl group, and a mono- or di- lower alkylaminocarbonyl group.

Examples of the lower alkoxyl groups include a C1-C6 alkoxyl group, and specific examples include a methoxyl group, an ethoxyl group, a propoxyl group, an isopropoxyl group, a butoxyl group, a secondary butoxyl group, and a tertiary butoxyl group.

Examples of the alkoxycarbonyl groups include a methoxycarbonyl group, an ethoxycarbonyl group, a propoxycarbonyl group, and a butoxycarbonyl group.

Examples of the carboxyalkyl groups include a carboxymethyl group, a carboxyethyl group, and a carboxypropyl group.

Examples of the alkoxycarbonylalkyl groups include a methoxycarbonylmethyl group, an ethoxycarbonylmethyl group, a proxycarbonylmethyl group, a methoxycarbonylethyl group, ethoxycarbonylethyl group, a methoxycarbonylpropyl group, and an ethoxycarbonylpropyl group.

Examples of the carboxyalkoxyl groups include a carboxymethoxyl group, a carboxyethoxyl group, and a carboxypropoxyl group. Examples of the alkoxycarbonylalkoxyl group include a methoxycarbonylmethoxyl group, an ethoxycarbonylmethoxyl group, a propoxycarbonylmethoxyl group, a methoxycarbonylethoxyl group, and an ethoxycarbonylethoxyl group.

Examples of the hydroxyalkyl groups include a hydroxymethyl group, a hydroxyethyl group, a hydroxypropyl group, and a hydroxybutyl group. Examples of the C1-C4 alkylene groups include a methylene group, an ethylene group, a trimethylene group, and a tetramethylene group.

Examples of the mono- or di- lower alkylaminocarbonyl groups include mono-lower alkylaminocarbonyl groups such as a methylaminocarbonyl group, an ethyaminocarbonyl group, a propylaminocarbonyl group, an isopropylaminocarbonyl group, a butylaminocarbonyl group, an isobutylaminocarbonyl group, a pentylaminocarbonyl group, an isopentylaminocarbonyl group, a hexylaminocarbonyl group, and an isohexylaminocarbonyl group. Examples of the di-alkylaminocarbonyl groups include symmetric dialkylaminocarbonyl group having two lower alkyl groups as substituents such as a dimethylaminocarbonyl group, a diethylaminocarbonyl group, a dipropylaminocarbonyl group, a diisopropylaminocarbonyl group, a dibutylaminocarbonyl group, and a dipentylaminocarbonyl group; and asymmetric dialkylaminocarbonyl groups having two different lower alkyl groups as substituent such as an ethylmethylaminocarbonyl group, a methylpropylaminocarbonyl group, an ethylpropylaminocarbonyl group, a butylmethylaminocarbonyl group, a butylethylaminocarbonyl group, and a butylpropylaminocarbonyl group.

Examples of the lower alkylsulfonyl groups include a methylsulfonyl group, an ethylsulfonyl group, a propylsulfonyl group, an isopropylsulfonyl group, a butylsulfonyl group, an isobutylsulfonyl group, a pentylsulfonyl group, an isopentylsulfonyl group, a hexylsulfonyl group, and an isohexylsulfonyl group.

With regard to the mono- or di-lower alkylaminothiocarbonyl groups, examples of the monoloweralkylaminothiocarbonyl groups include a methylaminothiocarbonyl group, an ethylaminothiocarbonyl group, a propylaminothiocarbonyl group, an isopropylaminothiocarbonyl group, a butylaminothiocarbonyl group, an isobutylaminothiocarbonyl group, a pentylaminothiocarbonyl group, an isopentylaminothiocarbonyl group, a hexylaminothiocarbonyl group, or an isohexylaminothiocarbonyl group. Examples of the dialkylaminothiocarbonyl group include symmetric dialkylaminothiocarbonyl group di-substituted by lower alkyl groups such as a dimethylaminothiocarbonyl group, a diethylaminothiocarbonyl group, a dipropylaminothiocarbonyl group, a diisopropylaminothiocarbonyl group, a dibutylaminothiocarbonyl group, or a dipentylaminothiocarbonyl group and asymmetric dialkylaminotiocarbonyl groups di-substituted by different lower alkyl groups such as an ethylmethylaminothiocarbonyl group, a methylpropylaminothiocarbonyl group, an ethylpropylaminothiocarbonyl group, a butylmethylaminothiocarbonyl group, a butylethylaminothiocarbonyl group, or a butylpropylaminothiocarbonyl group.

Examples of the lower alkanoyl groups include a formyl group, an acetyl group, a propionyl group, a butyryl group, an isobutyryl group, a valeryl group, an isovaleryl group, a pivaloyl group, and a hexanoyl group. Of these, an acetyl group, a propionyl group and a butyryl group are preferred, with an acetyl group and a propionyl group being more preferred. The lower alkanoyl group may have a substituent.

Examples of groups which may serve as a substituent for the alkanoyl group include a halogen atom, a carboxyl group, a carbamoyl group, an amino group, a cyano group, a nitro group, a lower alkanoyl group, a lower alkoxyl group, a lower alkoxycarbonyl group, a mono- or di- lower alkylamino group, an aryl group, an aralkyloxy group, an aryloxy group, a mercapto group, a lower alkylthio group, a lower alkylthiocarbonyl group, a hydroxyl group, a carbamoyl group, or a mono- or di- lower alkylaminocarbonyl group.

Examples of the aryl groups include a phenyl group, a naphthyl group, a biphenyl group, and an anthryl group. The aryl group may have a substituent.

Examples of the heteroaryl groups include a furyl group, a thienyl group, a pyrolyl group, an imidazolyl group, a pyrazolyl group, an isothiazolyl group, an isoxazolyl group, a pyridyl group, a pyrimidinyl group, a quinolyl group, an isoquinolyl group, a quinazolinyl group, a quinolidinyl group, a quinoxalinyl group, a cinnolinyl group, a benzimidazolyl group, an imidazopyridyl group, a benzofuranyl group, a naphthylidinyl group, a 1,2-benzoisoxazolyl group, a benzoxazolyl group, a benzothiazolyl group, an oxazolopyridyl group, an isothiazolopyridyl group, and a benzothienyl group. Of these, a furyl group, a thienyl group, a pyrolyl group, an imidazolyl group, and a pyridyl group are preferred. The aryl group may have a substituent.

Examples of groups which may serve as a substituent of these aryl or heteroaryl groups include a halogen atom, a carboxyl group, an amino group, a cyano group, a nitro group, a hydroxyl group, a lower alkoxyl group, a lower alkoxycarbonyl group, a mono- or di-lower alkylamino group, a lower alkanoyl group, and a lower alkyl group optionally having a substituent.

Preferably, the saturated or unsaturated 5- or 6-membered heterocyclic group is a heterocyclic group having 1 or 2 nitrogen or oxygen atoms. Specific examples of the heterocycles include pyrrolidine, piperidine, imidazoline, piperazine, tetrahydrofuran, hexahydropyrimidine, pyrrole, imidazole, pyrazine, pyrrolidinone, piperidinone, and morpholine. Of these, pyrrolidine and piperidine are particularly preferred. Examples of the saturated or unsaturated cyclic hydrocarbon groups include a cyclopentyl group and a cyclohexyl group. Examples of the aminoalkyl groups include an aminomethyl group, an aminoethyl group, and an aminopropyl group.

The heterocyclic groups and cyclic hydrocarbon groups may have a substituent. Examples of the groups which may serve as a substituent of the heterocyclic groups and cyclic hydrocarbon groups include a lower alkyl group, a lower alkanoyl group, a carbamoyl group, a monoalkylcarbamoyl group, a dialkylcarbamoyl group, a formimidoyl group, an alkanoimidoyl group, a benzimidoyl group, a carboxyl group, an alkoxycarbonyl group, a carboxyalkyl group, an alkylcarbonylalkyl group, an aminoalkyl group, an alkanoylamino group, an alkanoylaminoalkyl group, an imino group, and an alkoxycarbonylimino group.

Examples of groups which may serve as a substituent of the amino group and aminoalkyl group include a lower alkyl group, a pyrrolidinyl group, a pyrazyl group, a carbamoyl group, a monoalkylcarbamoyl group, a dialkylcarbamoyl group, a lower alkanoyl group, a formimidoyl group, an alkanoimidoyl group, a benzimidoyl group, and an alkoxycarbonyl group.

The above-described groups such as an alkyl group, an alkoxyl group, and an alkanoyl group and moieties in the substituents such as an alkyl moiety, an alkoxyl moiety, or an alkanoyl moiety preferably have 1 to 6 carbon atoms.

Particularly preferable examples of the group represented by the following formula: 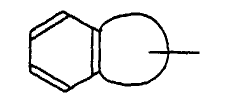 include a group selected from among benzofuranyl, benzimidazolyl, indolyl, benzothienyl, benzothiazolyl, naphthyl, and tetrahydronaphthyl.

The aromatic amidine derivatives represented by formula (1) according to the present invention, salts of the derivatives, solvates of the derivatives, and solvates of the salts of the derivatives may have an asymmetric carbon atom. In this case, optical isomers, stereoisomers, and mixtures thereof attributed to the asymmetric carbon atom are all within the scope of the present invention.

In the present invention, among the above-described aromatic amidine derivatives, salts of the derivatives, solvates of the derivatives, and solvates of the salts of the derivatives, preferred drugs are aromatic amidine derivatives represented by formula (1), salts of the derivatives, solvates of the derivatives, and solvates of the salts of the derivatives. Of these, the following compounds are particularly preferred:
2-[4-[((3S)-1-acetimidoyl-3-pyrrolidinyl)oxy]phenyl]-3-(7-amidino-2-naphthyl)propionic acid,
(+)-2-[4-[((3S)-1-acetimidoyl-3-pyrrolidinyl)oxy]phenyl]-3-(7-amidino-2-naphthyl)propionic acid,
(2S)-2-[4-[((3S)-1-acetimidoyl-3-pyrrolidinyl)oxy]phenyl]-3-(7-amidino-2-naphthyl)propionic acid,
(2R)-2-[4-[((3R)-1-acetimidoyl-3-pyrrolidinyl)oxy]phenyl]-3-(7-amidino-2-naphthyl)propionic acid,
2-[4-[(1-acetimidoyl-4-piperidyl)oxy]phenyl]-3-(7-amidino-2-naphthyl)propionic acid,
(+)-2-[4-[(1-acetimidoyl-4-piperidyl)oxy]phenyl]-3-(7-amidino-2-naphthyl)propionic acid,
2-[4-[(1-acetimidoyl-4-piperidyl)oxy]phenyl]-3-(5-amidinobenzo[b]thien-2-yl)propionic acid,
2-[4-[((2S)-1-acetimidoyl-2-pyrrolidinyl)methoxy]phenyl]-3-(5-amidinobenzo[b]thien-2-yl)propionic acid,
(+)-2-[4-[((2S)-1-acetimidoyl-2-pyrrolidinyl)methoxy]phenyl]-3-(5-amidinobenzo[b]thien-2-yl)propionic acid,
3-(4-[((3S)-1-acetimidoyl-3-pyrrolidinyl)oxy]phenyl]-4-(5-amidinobenzo[b]thien-2-yl)butyric acid,
2-[4-[((3S)-1-acetimidoyl-3-pyrrolidinyl)oxy]phenyl]-3-(6-amidino-1-ethyl-2-indolyl)propionic acid,
2-[4-[((3R)-1-acetimidoyl-3-pyrrolidinyl)oxy]phenyl]-3-(6-amidino-1-ethyl-2-indolyl)propionic acid,
2-[4-[(1-acetimidoyl-4-piperidyl)oxy]phenyl]-3-(6-amidino-1-ethyl-2-indolyl)propionic acid,
N-[4-[(1-acetimidoyl-4-piperidyl)oxy]phenyl]-N-[(7-amidino-2-naphthyl)methyl]-N'-methylsulfamide,
ethyl N-[N-4-[(1-acetimidoyl-4-piperidyl)oxy]phenyl]-N-[(7-amidino-2-naphthyl)methyl]sulfamoyl]carbamate,
4-[N-4-[(1-acetimidoyl-4-piperidyl)oxy]phenyl]-N-[(7-amidino-2-naphthyl)methyl]sulfamoyl]benzoic acid,
N-[4-[(1-acetimidoyl-4-piperidyl)oxy]phenyl]-N-[(7-amidino-2-naphthyl)methyl]sulfamoylacetic acid,
ethyl N-[N-[4-[(1-acetimidoyl-4-piperidyl)oxy]phenyl]-N-[(7-amidino-2-naphthyl)methyl]sulfamoyl]glycinate,
N-[N-4-[(1-acetimidoyl-4-piperidyl)oxy]phenyl]-N-[(7-amidino-2-naphthyl)methyl]sulfamoyl]-N-ethoxycarbonylglycine, and
N-[N-4-[(1-acetimidoyl-4-piperidyl)oxy]phenyl]-N-[(7-amidino-2-naphthyl)methyl]sulfamoyl]glycine.

Particularly preferred ones are:
(2S)-2-[4-[((3S)-1-acetimidoyl-3-pyrrolidinyl)oxy]phenyl]-3-(7-amidino-2-naphthyl)propionic acid,
(+)-2-[4-[(1-acetimidoyl-4-piperidyl)oxy]phenyl]-3-(7-amidino-2-naphthyl)propionic acid,
(+)-2-[4-[((2S)-1-acetimidoyl-2-pyrrolidinyl)methoxy]phenyl]-3-(5-amidinobenzo[b]thien-2-yl)propionic acid,
ethyl N-[N-[4-[(1-acetimidoyl-4-piperidyl)oxy]phenyl]-N-[(7-amidino-2-naphthyl)methyl]sulfamoyl]glycinate,
N-[N-4-[(1-acetimidoyl-4-piperidyl)oxy]phenyl]-N-[(7-amidino-2-naphthyl)methyl]sulfamoyl]glycine, and
N-[4-[(1-acetimidoyl-4-piperidyl)oxy]phenyl]-N-[(7-amidino-2-naphthyl)methyl]sulfamoylacetic acid.

Furthermore, the following compounds are also preferred:
(2S)-2-[4-[((3S)-1-acetimidoyl-3-pyrrolidinyl)oxy]phenyl]-3-(7-amidino-2-naphthyl)propionic acid hydrochloride pentahydrate, (+)-2-[4-[(1-acetimidoyl-4-piperidyl)oxy]phenyl]-3-(7-amidino-2-naphthyl)propionic acid dihydrochloride,
(+)-2-[4-[((2S)-1-acetimidoyl-2-pyrrolidinyl)methoxy]phenyl]-3-(5-amidinobenzo[b]thien-2-yl)propionic acid dihydrochloride, ethyl N-[N-[4-[(1-acetimidoyl-4-piperidyl)oxy]phenyl]-N-[(7-amidino-2-naphthyl)methyl]sulfamoyl]glycinate dihydrochloride, N-[N-4-[(1-acetimidoyl-4-piperidyl)oxy]phenyl]-N-[(7-amidino-2-naphthyl)methyl]sulfamoyl]glycine dihydrochloride, and N-[4-[(1-acetimidoyl-4-piperidyl)oxy]phenyl]-N-[(7-amidino-2-naphthyl)methyl]sulfamoylacetic acid dihydrochloride.

The term "lipophilic substance" in the present invention refers to a substance having low affinity to water, and examples include higher alcohols, fatty acids, fatty acid salts, esters formed between a fatty acid and an alcohol or a polyhydric alcohol. The substance exists ubiquitously in natural animals and plants as a component of, for example, fat and oil or lipid. Thus, examples of the lipophilic substances in the present invention also include natural oil and fat and natural lipid containing the substances.

The higher alcohols, fatty acids, fatty acid salts, esters of a fatty acid and an alcohol or a polyhydric alcohol which serve as the lipophilic substances in the present invention will next be described.

Examples of lipophilic higher alcohols include C6-C24 linear, branched, or cyclic alcohols. Specific examples thereof include cetyl alcohol, stearyl alcohol, isostearyl alcohol, 2-octyldodecanol, and 2-hexyldecanol.

Examples of lipophilic fatty acids include C6-C13 saturated or unsaturated medium-chain fatty acids and C14-C22 saturated or unsaturated long-chain fatty acids. Specific examples of medium-chain fatty acids include caproic acid, enanthic acid, caprylic acid, pelargonic acid, capric acid, undecylic acid, undecylenic acid, and lauric acid. Examples of long-chain fatty acids include myristic acid, pentadecanoic acid, palmitic acid, palmitolic acid, margaric acid, stearic acid, oleic acid, linoleic acid, linolenic acid, nonadecanoic acid, arachic acid, eicosenic acid, arachidoic acid, and behenic acid.

Examples of fatty acid salts include alkali metal salts and alkaline earth metal salts of the above-described fatty acids. Examples of alkali metal salts include sodium salts and potassium salts, and examples of the alkaline earth metal salts include magnesium salts and calcium salts. Specific examples include sodium caproate, sodium caprylate, sodium myristate, and sodium palmitate.

Examples of esters between a fatty acid and an alcohol or a polyhydric alcohol include esters formed between the above-described fatty acids and an alcohol or a polyhydric alcohol. Examples of alcohols include a C1-C8 linear, branched, or cyclic monohydric alcohol such as methanol, ethanol, isopropanol, or butanol.

Specific examples of esters between the fatty acids and monohydric alcohols include butyl myristate, isopropyl palmitate, methyl stearate, and ethyl linoleate.

No particular limitation is imposed on the polyhydric alcohol so long as it is a compound having two or more alcoholic hydroxyl groups, and examples include ethylene glycol, polyethylene glycol, propylene glycol, polypropylene glycol, glycerin, polyglycerin, and sorbitan. Of these, glycerin and polyglycerin are more preferable.

Examples of the esters between fatty acid and polyhydric alcohol include fatty acid glycerin esters, fatty acid glycerin acetic acid esters, fatty acid glycerin lactic acid esters, fatty acid glycerin citric acid esters, fatty acid glycerin succinic acid esters, fatty acid glycerin diacetyl tartaric acid ester esters, fatty acid polyglycerin esters, fatty acid ethylene glycol esters, fatty acid propylene glycol esters, fatty acid polyethylene glycol esters, and fatty acid polypropylene glycol esters.

The esters between fatty acid and polyhydric alcohol are formed by linking a fatty acid with one or more hydroxyl group contained in the polyhydric alcohol to form ester linkage. When a plurality of hydroxyl groups form ester linkage with fatty acids, the fatty acid may be identical to or different from one another. Medium-chain or long-chain fatty acids are preferable as the fatty acids which form the polyhydric alcohol esters.

Among the esters between fatty acid and polyhydric alcohol, esters between fatty acid and glycerin or polyglycerin are preferred, with esters between the medium-chain or long-chain fatty acid and glycerin or polyglycerin being particularly preferred.

Specific examples include glycerin caproate, glycerin caprylate, glycerin caprate, glycerin palmitate, glycerin stearate, and hydrogenated tallow fatty acid glycerides. Of these, glycerin caprylate and glycerin caprate are particularly preferred.

With regard to the lipophilic substance in the present invention, esters between fatty acid and polyhydric alcohol are preferred, with esters between fatty acid and glycerin or polyglycerin being more preferred and esters between fatty acid and glycerin being still more preferred. Of these, esters between medium-chain fatty acid and glycerin and esters between long-chain fatty acid and glycerin are preferable, with triesters between medium-chain fatty acid and glycerin, monoesters between long-chain fatty acid and glycerin, and triesters between long-chain fatty acid and glycerin.

Specifically, preferable examples include glycerin tricaprylate, glycerin tricaprate, hydrogenated tallow fatty acid triglycerides, glycerin tripalmitate, and glycerin monostearate. Particularly preferred examples include mixtures of glycerin tricaprylate and glycerin tricaprate; mixtures of glycerin tricaprylate, glycerin tricaprate, and hydrogenated tallow fatty acid triglycerides and mixtures of glycerin tricaprylate, glycerin tricaprate, and glycerin monostearate.

The fatty acid composition of these lipophilic substances is not purified, since they are generally produced by use of natural materials originating from animals or vegetables. These lipophilic substances are also advantageously used for achieving an object of the present invention. In the present invention, the lipophilic substances may be used singly or in combination of two or more species.

The content of the lipophilic substance (compositional proportion) in the pharmaceutical composition according to the present invention may be modified appropriately in accordance with the type of the basic drug, and it is preferably 0.1-25 times, more preferably 0.5-15 times by weight based on the basic drug.

The term "cyclodextrin" in the present description refers to a group of cyclodextrin and derivatives thereof. Examples of cyclodextrin include α-cyclodextrin, β-cyclodextrin, γ-cyclodextrin, and derivatives thereof.

In the present invention, water-soluble cyclodextrins are preferable. Examples include water-soluble β-cyclodextrin, water-soluble γ-cyclodextrin, and water-soluble derivatives thereof. Specific examples include β-cyclodextrin, γ-cyclodextrin, hydroxypropyl-β-cyclodextrin, hydroxypropyl-γ-cyclodextrin, methyl-β-cyclodextrin, methyl-γ-cyclodextrin, dimethyl-β-cyclodextrin, dimethyl-γ-cyclodextrin, sulfobutyl-β-cyclodextrin, and sulfobutyl-γ-cyclodextrin. In the present invention, β-cyclodextrin, γ-cyclodextrin, hydroxypropyl-β-cyclodextrin, hydroxypropyl-γ-cyclodextrin, methyl-β-cyclodextrin, and sulfobutyl-β-cyclodextrin are preferred. Of these, β-cyclodextrin is particularly preferred.

The content of the cyclodextrin in the pharmaceutical composition according to the present invention may be modified appropriately in accordance with the type of basic drugs, and the content (compositional proportion) is preferably 0.1-10 mol, more preferably 0.3-7 mol to 1 mol of the basic drug.

In the present invention, the lipophilic substance and the cyclodextrin are particularly preferably used in combination in that peroral absorption of a basic drug increases.

In the present invention, the following pharmaceutical compositions are particularly preferable:
a pharmaceutical composition containing (2S)-2-[4-[((3S)-1-acetimidoyl-3-pyrrolydinyl)oxy]phenyl]-3-(7-amidino-2-naphthyl)propionic acid hydrochloride pentahydrate, a lipophilic substance, and cyclodextrin;
a pharmaceutical composition containing (+)-2-[4-[(1-acetimidoyl-4-piperidyl)oxy]phenyl]-3-(7-amidino-2-naphthyl)propionic acid dihydrochloride, a lipophilic substance, and cyclodextrin;
a pharmaceutical composition containing (+)-2-[4-[((2S)-1-acetimidoyl-2-pyrrolydinyl)methoxy]phenyl]-3-(5-amidinobenzo[b]thien-2-yl)propionic acid dihydrochloride, a lipophilic substance, and cyclodextrin;
a pharmaceutical composition containing ethyl N-[N-[4-[(1-acetimidoyl-4-piperidyl)oxy]phenyl]-N-[(7-amidino-2-naphthyl)methyl]sulfamoyl]glycinate dihydrochloride, a lipophilic substance, and cyclodextrin;
a pharmaceutical composition containing N-[N-4-[(1-acetimidoyl-4-piperidyl)oxy]phenyl]-N-[(7-amidino-2-naphthyl)methyl]sulfamoyl]glycine dihydrochloride, a lipophilic substance, and cyclodextrin; and
a pharmaceutical composition containing N-[4-[(1-acetimidoyl-4-piperidyl)oxy]phenyl]-N-[(7-amidino-2-naphthyl)methyl]sulfamoylacetic acid dihydrochloride a lipophilic substance, and cyclodextrin.

Of these, a pharmaceutical composition containing (2S)-2-[4-[((3S)-1-acetimidoyl-3-pyrrolydinyl)oxy]phenyl]-3-(7-amidino-2-naphthyl)propionic acid hydrochloride pentahydrate, a lipophilic substance, and cyclodextrin is particularly preferred.

The pharmaceutical composition according to the present invention may be formed into any physical form so long as it can be administered perorally. Examples of the physical form include powders, tablets, capsules, liquids, suspensions, and emulsions.

When the pharmaceutical composition according to the present invention contains a water-soluble basic drug and a lipophilic substance, examples of the possible forms of the drug include semi-solid formulations such as emulsions, suspensions, and gels. Such semi-solid preparations may be filled in microcapsules, soft capsules, hard capsules, etc., to thereby form capsule products. Capsules are preferable because they are easily taken by patients.

The formulations may be prepared through a generally known method, and pharmaceutical additives such as excipients, disintegrators, binders, lubricants, fluidisers, dispersants, suspending agents, emulsifiers, preservatives, and stabilizers may be added as desired.

The present invention will next be described in detail by way of examples, which should not be construed as limiting the invention thereto.

### Examples

### [Comparative Example 1]

(2S)-2-[4-[((3S)-1-Acetimidoyl-3-pyrrolidinyl)oxy]phenyl]-3-(7-amidino-2-naphthyl)propionic acid hydrochloric acid salt pentahydrate (hereinafter referred to as compound A) (0.5 mg) was dissolved in physiological saline (0.5 ml). The solution was perorally administered to rats (SD male rats, 8 weeks old, body weight: 230-270 g, n=7) without anesthesia through a peroral probe. Blood were collected from jugular vein after 0.5, 1, 2, 4, or 8 hours following administration under ether anethesia. Plasma concentration of compound A was determined by high performance liquid chromatography (HPLC). From the time-course profile in plasma concentration of compound A, the area under the plasma concentration-time curve (AUC) and maximum concentration in plasma (Cmax) were obtained.

As described in Japanese Patent Application Laid-Open (*kokai*) No. 5-208946, compound A inhibits blood coagulation factor X and thus is useful as an anticoagulant or for the prevention and/or treatment of thrombus.

### [Example 1]

Compound A (0.5 mg) was mixed with γ-cyclodextrin (7.26 mg; i.e., in an amount 5 times that of compound A on a molar basis) and the resultant mixture was dissolved in saline (0.5 ml). In a manner similar to that of Comparative Example 1, the solution was perorally administered to rats, and AUC and Cmax were determined through measurement of the concentration of unchanged substance in plasma.

### [Example 2]

The procedure of Example 1 was repeated except that hydroxypropyl-β-cyclodextrin (6.72 mg) was used instead of γ-cyclodextrin (7.26 mg) to thereby obtain a solution. In a manner similar to that of Comparative Example 1, the solution was perorally administered to rats, and AUC and Cmax were determined through measurement of the concentration of unchanged substance in plasma.

### [Example 3]

γ-cyclodextrin (14.52 mg) was dissolved in saline (0.5 ml) to thereby obtain a solution. The solution was perorally administered to rats in a manner similar to that of Comparative Example 1. After 20 minutes, a solution prepared by the following method was perorally administrated to the same rats: Compound A (0.5 mg) was mixed with γ-cyclodextrin (7.26 mg; i.e., in an amount 5 times that of compound A on a molar basis), and the resultant mixture was dissolved in saline (0.5 ml). After elapse of 40 additional minutes, γ-cyclodextrin (14.52 mg) dissolved in saline (0.5 ml) was perorally administrated to the same rats. AUC and Cmax were determined through measurement of the concentration of unchanged substance in plasma.

### [Example 4]

The procedure of Example 3 was repeated except that hydroxypropyl-β-cyclodextrin was used instead of γ-cyclodextrin to thereby obtain a solution. The solution was perorally administered to rats, and AUC and Cmax were determined through measurement of the concentration of unchanged substance in plasma.

### [Example 5]

The procedure of Example 3 was repeated except that β-cyclodextrin, instead of γ-cyclodextrin, was homogeneously dispersed in a state of suspension in the saline to thereby obtain a solution. The solution was perorally administered to rats, and AUC and Cmax were determined through measurement of the concentration of unchanged substance in plasma.

AUC and Cmax obtained in Comparative Example 1 and Examples 1 to 5 are shown in Table 1.

**Table 1**

| (Average ± S.D.) | | |
|---|---|---|
| | AUC (hr^{.}µg/ml) | Cₘₐₓ (µg/ml) |
| Comparative Example 1 | 0.24 ± 0.24 | 0.12 ± 0.10 |
| Example 1 | 0.46 ± 0.20 | 0.21 ± 0.08 |
| Example 2 | 0.52 ± 0.38 | 0.27 ± 0.20 |
| Example 3 | 0.81 ± 0.32 | 0.27 ± 0.08 |
| Example 4 | 0.75 ± 0.22 | 0.31 ± 0.13 |
| Example 5 | 0.91 ± 0.45 | 0.33 ± 0.14 |

As shown in Table 1, the pharmaceutical composition of the present invention for oral administration, which comprises the basic drug and cyclodextrin or the like, showed improved peroral absorption compared to the case for oral administration only the basic drug.

### [Example 6]

The procedure of Comparative Example 1 was repeated except that compound A (0.5 mg) was suspended in 0.5 ml of medium-chained fatty acid triglyceride (mixture of tricapryl acid glycerin ester and tricapric acid glycerin ester) [trade name: Migriol 812 neutral oil; Mitsuba Trading Company] to thereby obtain a solution, and rats (SD male rats, 8 weeks old, body weight: 230-270 g, n=5) were used. Bloods were collected after oral administration of the solution to rats in a manner similar to comparative Example 1. And AUC and Cmax were determined through measurement of compound A in plasma.

### [Example 7]

Compound A (0.5 mg) was mixed with β-cyclodextrin (6.38 mg; i.e., in an amount 5 times that of compound A on a molar basis) and the resultant mixture was suspended in 0.5 ml of medium-chained fatty acid triglyceride [trade name: Migriol 812 neutral oil (mixture of tricapryl acid glycerin ester and tricapric acid glycerin ester); Mitsuba Trading Company]. In a manner similar to that of Example 6, the solution was perorally administered to rats, bloods were collected, and AUC and Cmax were determined through measurement of compound A in plasma.

### [Example 8]

The procedure of Example 7 was repeated except that γ-cyclodextrin (7.29 mg) was used instead of β-cyclodextrin to thereby obtain a solution. In the same manner as that of Example 6, the solution was perorally administered to rats, bloods were collected, and AUC and Cmax were determined through measurement of compound A in plasma.

### [Example 9]

The procedure of Example 7 was repeated except that olive oil was used instead of medium-chained fatty acid triglyceride. In the same manner as that of Example 6, the solution was perorally administered to rats, bloods were collected, and AUC and Cmax were determined through measurement of compound A in plasma.

### [Example 10]

The procedure of Example 7 was repeated except that a mixture of medium-chained fatty acid triglyceride and tripalmitic acid glycerin ester (medium-chained fatty acid triglyceride : tripalmitic acid glycerin ester = 9:1 (weight ratio)) was used instead of medium-chained fatty acid triglyceride to thereby obtain a solution. In the same manner as that of Example 6, the solution was perorally administered to rats, bloods were collected, and AUC and Cmax were determined through measurement of compound A in plasma.

AUC and Cmax obtained in Examples 6 to 10 are shown in Table 2.

**Table 2**

| (average ± S.D.) | | |
|---|---|---|
| | AUC (hr^{.}µg/ml) | Cₘₐₓ (µg/ml) |
| Example 6 | 1.03 ± 0.53 | 0.22 ± 0.13 |
| Example 7 | 1.66 ± 0.50 | 0.39 ± 0.16 |
| Example 8 | 1.66 ± 0.90 | 0.41 ± 0.25 |
| Example 9 | 1.06 ± 0.36 | 0.25 ± 0.12 |
| Example 10 | 0.99 ± 0.32 | 0.24 ± 0.08 |

### [Comparative Example 2]

Compound A was dissolved in saline, and the solution was perorally administered to monkeys (female, wild cynomolgus monkeys, body weight: 2.4-3.3 kg, n=4) so as to attain a dosage of 2 mg/ml/kg, without anesthesia and through an oral stomach tube. Bloods were collected from the femoral vein 0.5, 1, 2, 3, 4, or 8 hours following administration. Plasma concentration of compound A was determined by radioimmunoassay. From the time-course profile in plasma concentration of compound A, AUC and Cmax were obtained.

### [Example 11]

Compound A (6 mg) was mixed with β-cyclodextrin (62.8 mg; i.e., in an amount 4.1 times that of compound A on a molar basis) and the resultant mixture was suspended in 70.3 mg of medium-chained fatty acid triglyceride [trade name: Migriol 812 neutral oil; Mitsuba Trading Company]. The resultant suspension was filled in gelatin capsules (No. 5). The capsulated suspension was perorally administered to monkeys (one capsule/animal). In a manner similar to that of Comparative Example 2, bloods were collected from the monkeys, and AUC and Cmax were determined through measurement of compound A in plasma.

### [Example 12]

Compound A (6 mg) was mixed with β-cyclodextrin (30.6 mg; i.e., in an amount 2.0 times that of compound A on a molar basis) and the resultant mixture was suspended in 89.4 mg of medium-chained fatty acid triglyceride [trade name: Migriol 812 neutral oil; Mitsuba Trading Company]. The resultant suspension was filled in seamless microcapsules (diameter: 2 mm⌀). The microcapsulated suspension was perorally administered to monkeys (6 mg compound A per animal). In a manner similar to that of Comparative Example 2, bloods were collected from the monkeys, and AUC and Cmax were determined through measurement of compound A in plasma.

### [Example 13]

Example 11 was repeated except that a mixture (70.3 mg) of medium-chained fatty acid triglyceride and hydrogenated tallow fatty acid triglyceride [trade name: Trifat T52; Nikko Chemical Company] was used (medium-chained fatty acid triglyceride : hydrogenated tallow fatty acid triglyceride = 9 : 1 by weight) instead of medium-chained fatty acid triglyceride. In a manner similar to that of Example 11, microcapsules were perorally administered to monkeys. Bloods were collected, and AUC and Cmax were determined through measurement of compound A in plasma.

### [Example 14]

Example 11 was repeated except that a mixture (70.3 mg) of medium-chained fatty acid triglyceride and monostearic acid gliceryl ester [trade name: CUTINA GMS-RP; Henkel Company] was used (medium-chained fatty acid triglyceride : monostearic acid glyceryl ester = 19 : 1 by weight) instead of medium-chained fatty acid triglyceride. In a manner similar to that of Example 11, microcapsules were perorally administered to monkeys. Bloods were collected, and AUC and Cmax were determined through measurement of compound A in plasma.

### [Example 15]

Example 14 was repeated by use of a mixture (70.3 mg) of medium-chained fatty acid triglyceride and monostearic acid gliceryl ester (medium-chained fatty acid triglyceride : monostearic acid glyceryl ester = 39:1 by weight instead of 19:1). In a manner similar to that of Example 11, the sample was perorally administered to monkeys. Bloods were collected, and AUC and Cmax were determined through measurement of compound A in plasma.

The AUC and Cmax obtained from the above-described Comparative Example 2, and Examples 11 to 15 are shown in Table 3.

**Table 3**

| (Average ± S.D.) | | |
|---|---|---|
| | AUC (hr^{.}µg/ml) | Cₘₐₓ (µg/ml) |
| Comparative Example 2 | 0.28 ± 0.12 | 0.08 ± 0.04 |
| Example 11 | 0.54 ± 0.27 | 0.19 ± 0.09 |
| Example 12 | 0.39 ± 0.99 | 0.13 ± 0.04 |
| Example 13 | 0.62 ± 0.18 | 0.23 ± 0.07 |
| Example 14 | 0.58 ± 0.15 | 0.31 ± 0.13 |
| Example 15 | 0.99 ± 0.68 | 0.48 ± 0.32 |

As is apparent from Tables 1, 2, and 3, the pharmaceutical composition of the present invention which comprises a basic drug and a lipophilic substance or cyclodextrin, or the pharmaceutical composition which comprises the basic drug, the lipophilic substance, and cyclodextrin showed improved peroral absorption as compared with the case in which a basic drug is administered alone (Comparative Examples 1 and 2).

## Claims

1. A pharmaceutical composition for oral administration which comprises (a) a basic drug and (b) a lipophilic substance and/or a cyclodextrin.

2. A composition according to claim 1, wherein the basic drug (a) is an aromatic amidine derivative, a salt of the derivative, a solvate of the derivative, or a solvate of the salt of the derivative.

3. A composition according to claim 1 or 2, wherein the basic drug (a) is an aromatic amidine derivative of formula (1), a salt of the derivative, a solvate of the derivative, or a solvate of the salt of the derivative: 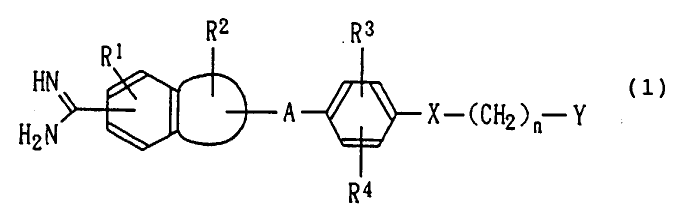
[wherein R¹ represents a hydrogen atom or a lower alkoxyl group;
R² represents a hydrogen atom, a lower alkyl group, a lower alkoxyl group, a carboxyl group, an alkoxycarbonyl group, a carboxyalkyl group, or an alkoxycarbonylalkyl group;
R³ represents a hydrogen atom, a carboxyl group, an alkoxycarbonyl group, a carboxyalkyl group, an alkoxycarbonylalkyl group, a carboxyalkoxyl group or an alkoxycarbonylalkoxyl group;
R⁴ represents a hydrogen atom, a halogen atom, an amino group, a cyano group, a nitro group, a hydroxyl group, a lower alkyl group, or a lower alkoxyl group;
n represents an integer 0 to 4; and
A represents a C1-C4 alkylene group optionally substituted by one or two hydroxyalkyl groups, carboxyl groups, alkoxycarbonyl groups, carboxyalkyl groups, or alkoxycarbonylalkyl groups, or a group represented by the following formula; 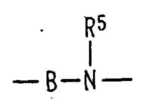
{wherein B represents a lower alkylene group or a carbonyl group and R⁵ represents a hydrogen atom or a group represented by formula -D-W-R⁶ (wherein D is a group represented by 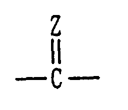
(wherein Z is an oxygen atom or a sulfur atom), or a group represented by 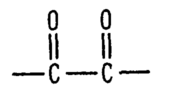 or a sulfonyl group;
W represents a single bond or a group represented by -NR⁷-(wherein R⁷ represents a hydrogen atom, a carbamoyl group, a lower alkoxycarbonyl group, a mono- or di-lower alkylaminocarbonyl group, a lower alkylsulfonyl group, a mono- or di-lower alkylaminothiocarbonyl group, a lower alkyl group which may have a substituent, or a lower alkanoyl group which may have a substituent);
R⁶ represents a hydroxyl group, a lower alkoxyl group, a lower alkyl group which may have a substituent, an aryl group which may have a substituent, or a heteroaryl group which may have a substituent)};
X represents a single bond, an oxygen atom, a sulfur atom, or a carbonyl group;
Y represents a saturated or unsaturated 5- or 6-membered heterocyclic or cyclic hydrocarbon group which may have a substituent, an amino group which may have a substituent, or an aminoalkyl group which may have a substituent; and the group represented 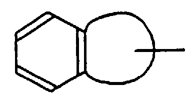 by represents a group selected from among indolyl, benzofuranyl, benzothienyl, benzimidazolyl, benzoxazolyl, benzothiazolyl, naphthyl, tetrahydronaphthyl, or indanyl].

4. A composition according to claim 3, wherein in formula (1) the group represented by 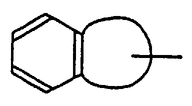 is selected from the group consisting of benzofuranyl, benzimidazolyl, indolyl, benzothienyl, benzothiazolyl, naphthyl, and tetrahydronaphthyl.

5. A composition according to claim 3 or 4, wherein in formula (1) the saturated or unsaturated 5- or 6-membered heterocyclic group has 1 or 2 nitrogen atoms or oxygen atoms.

6. A composition according to claim 4 or 5, wherein in formula (1) the saturated or unsaturated 5- or 6-membered heterocyclic group is a pyrrolidinyl group or a piperidyl group.

7. A composition according to any one of claims 3 through 6, wherein the aromatic amidine derivative, the salt of the derivative, the solvate of the derivative, or the solvate of the salt of the derivative is selected from the group consisting of the following compounds:
2-[4-[((3S)-1-acetimidoyl-3-pyrrolidinyl)oxy]phenyl]-3-(7-amidino-2-naphthyl)propionic acid,
(+)-2-[4-[((3S)-1-acetimidoyl-3-pyrrolidinyl)oxy]phenyl]-3-(7-amidino-2-naphthyl)propionic acid,
(2S)-2-[4-[((3S)-1-acetimidoyl-3-pyrrolidinyl)oxy]phenyl]-3-(7-amidino-2-naphthyl)propionic acid,
(2R)-2-[4-[((3R)-1-acetimidoyl-3-pyrrolidinyl)oxy]phenyl]-3-(7-amidino-2-naphthyl)propionic acid,
2-[4-[(1-acetimidoyl-4-piperidyl)oxy]phenyl]-3-(7-amidino-2-naphthyl)propionic acid,
(+)-2-[4-[(1-acetimidoyl-4-piperidyl)oxy]phenyl]-3-(7-amidino-2-naphthyl)propionic acid,
2-[4-[(1-acetimidoyl-4-piperidyl)oxy]phenyl]-3-(5-amidinobenzo[b]thien-2-yl)propionic acid,
2-[4-[((2S)-1-acetimidoyl-2-pyrrolidinyl)methoxy]phenyl]-3-(5-amidinobenzo[b]thien-2-yl)propionic acid,
(+)-2-[4-[((2S)-1-acetimidoyl-2-pyrrolidinyl)methoxy]phenyl]-3-(5-amidinobenzo[b]thien-2-yl)propionic acid,
3-[4-[((3S)-1-acetimidoyl-3-pyrrolidinyl)oxy]phenyl]-4-(5-amidinobenzo[b]thien-2-yl)butyric acid,
2-[4-[((3S)-1-acetimidoyl-3-pyrrolidinyl)oxy]phenyl]-3-(6-amidino-1-ethyl-2-indolyl)propionic acid,
2-[4-[((3R)-1-acetimidoyl-3-pyrrolidinyl)oxy]phenyl]-3-(6-amidino-1-ethyl-2-indolyl)propionic acid,
2-[4-[(1-acetimidoyl-4-piperidyl)oxy]phenyl]-3-(6-amidino-1-ethyl-2-indolyl)propionic acid,
N-[4-[(1-acetimidoyl-4-piperidyl)oxy]phenyl]-N-[(7-amidino-2-naphthyl)methyl]-N'-methylsulfamide,
ethyl N-[N-4-[(1-acetimidoyl-4-piperidyl)oxy]phenyl]-N-[(7-amidino-2-naphthyl)methyl]sulfamoyl]carbamate,
4-[N-4-[(1-acetimidoyl-4-piperidyl)oxy]phenyl]-N-[(7-amidino-2-naphthyl)methyl]sulfamoyl]benzoic acid,
N-[4-[(1-acetimidoyl-4-piperidyl)oxy]phenyl]-N-[(7-amidino-2-naphthyl)methyl]sulfamoylacetic acid,
ethyl N-[N-[4-[(1-acetimidoyl-4-piperidyl)oxy]phenyl]-N-[(7-amidino-2-naphthyl)methyl]sulfamoyl]glycinate,
N-[N-4-[(1-acetimidoyl-4-piperidyl)oxy]phenyl]-N-[(7-amidino-2-naphthyl)methyl]sulfamoyl]-N-ethoxycarbonylglycine, and
N-[N-4-[(1-acetimidoyl-4-piperidyl)oxy]phenyl]-N-[(7-amidino-2-naphthyl)methyl]sulfamoyl]glycine.

8. A composition according to any one of claims 3 through 7, wherein the aromatic amidine derivative, the salt of the derivative, the solvate of the derivative, or the solvate of the salt of the derivative is selected from the group consisting of the following compounds:
(2S)-2-[4-[((3S)-1-acetimidoyl-3-pyrrolidinyl)oxy]phenyl]-3-(7-amidino-2-naphthyl)propionic acid hydrochloride pentahydrate,
(+)-2-[4-[(1-acetimidoyl-4-piperidyl)oxy]phenyl]-3-(7-amidino-2-naphthyl)propionic acid dihydrochloride,
(+)-2-[4-[((2S)-1-acetimidoyl-2-pyrrolidinyl)methoxy]phenyl]-3-(5-amidinobenzo[b]thien-2-yl)propionic acid dihydrochloride, ethyl N-[N-[4-[(1-acetimidoyl-4-piperidyl)oxy]phenyl]-N-[(7-amidino-2-naphthyl)methyl]sulfamoyl]glycinate dihydrochloride, N-[N-4-[(1-acetimidoyl-4-piperidyl)oxy]phenyl]-N-[(7-amidino-2-naphthyl)methyl]sulfamoyl]glycine dihydrochloride, and N-[4-[(1-acetimidoyl-4-piperidyl)oxy]phenyl]-N-[(7-amidino-2-naphthyl)methyl]sulfamoylacetic acid dihydrochloride.

9. A composition according to any one of claims 1 through 8, wherein the lipophilic substance is selected from the group consisting of higher alcohols, fatty acids, fatty acid salts, and esters formed between a fatty acid and an alcohol or a polyhydric alcohol.

10. A composition according to any one of claims 1 through 9, wherein the lipophilic substance is an ester formed between a fatty acid and glycerin.

11. A composition according to any one of claims 1 through 10, wherein the lipophilic substance is an ester formed between a medium-chain fatty acid and glycerin.

12. A composition according to any one of claims 1 through 10, wherein the lipophilic substance is a mixture of an ester formed between a medium-chain fatty acid and glycerin, and a long-chain fatty acid and glycerin.

13. A composition according to any one of claims 1 through 10, wherein the lipophilic substance is a mixture of glycerin caprylate and glycerin caprate.

14. A composition according to any one of claims 1 through 10, wherein the lipophilic substance is a triester formed between a medium-chain fatty acid and glycerin.

15. A composition according to any one of claims 1 through 10 and claim 12, wherein the lipophilic substance is a mixture of glycerin tricaprylate, glycerin tricaprate, and glycerin monostearate.

16. A composition according to any one of claims 1 through 10 and claim 12, wherein the lipophilic substance is a mixture of glycerin tricaprylate, glycerin tricaprate, and hydrogenated tallow fatty acid triglyceride.

17. A composition according to any one of claims 1 through 16, wherein the cyclodextrin is a water-soluble cyclodextrin.

18. A composition according to any one of claims 1 through 17, wherein the cyclodextrin is a mixture of one or more members selected from the group consisting of β-cyclodextrin, γ-cyclodextrin, a water-soluble β-cyclodextrin derivative, and a water-soluble γ-cyclodextrin derivative.

19. A composition according to any one of claims 1 through 18, wherein the cyclodextrin is β-cyclodextrin.

20. An emulsion product which comprises a composition as described in any one of claims 1 through 19.

21. A suspension product which comprises a composition as described in any one of claims 1 through 19.

22. A capsule product which comprises a composition as described in any one of claims 1 through 19.

23. A capsule product which comprises a suspension product as described in claim 21.

24. A pharmaceutical composition for oral administration which comprises (2S)-2-[4-[((3S)-1-acetimidoyl-3-pyrrolydinyl)oxy]phenyl]-3-(7-amidino-2-naphthyl)propionic acid hydrochloride pentahydrate, glycerin tricaprylate, glycerin tricaprate, glycerin monostearate, and β-cyclodextrin.

25. A capsule product which comprises a composition as described in claim 24.
